# EUROPEAN PATENT APPLICATION

(11) **EP 2 353 537 A1**
(43) Date of publication of application: **10.08.2011**
(21) Application number: 09823357.0
(22) Date of filing: 02.11.2009
(51) Int. Cl.: A61B 19/00, A61B 1/00

(54) **ILLUMINATION SYSTEM FOR SURGICAL OPERATION**

(30) Priority: 31.10.2008 JP 2008281642
(71) Applicant: Limited Liability Company Japan Medical Creative, Saitama 351-0101 (JP)
(72) Inventor: KOYAMA, Isamu, Wako-shi Saitama 351-0101 (JP); TANAKA, Takeshi, Aki-gun Hiroshima 736-0032 (JP); HAYASHI, Shuro, Hatsukaichi-shi Hiroshima 738-0036 (JP)
(74) Representative: Delorme, Nicolas
(86) International application number: PCT/JP2009/005830
(87) International publication number: WO 2010/050244

(57) **Abstract**

A surgical lighting system includes: a lighting portion 10 having a light emitting diode 15; and a fixing needle 11 configured to fix the lighting portion 10 to a living organism to be operated on. After the lighting portion 10 is placed so that light of the lighting portion 10 is directly incident on an operative field, the needle 11 is inserted into biological tissue to fix the lighting portion 10 to the living organism.

## Description

### TECHNICAL FIELD

The present invention relates to surgical lighting systems that are used when operating on living organisms.

### BACKGROUND ART

Conventionally, shadowless lamps are known as an example of lighting equipment that is used for surgeries (see, e.g., Patent Document 1). Such a shadowless lamp includes a lamp unit having a multiplicity of lamps, and this lamp unit is normally attached to the ceiling of an operating room via a movable mechanism. When using the shadowless lamp, the position of the lamp unit is adjusted to meet the needs of a doctor who actually perform procedures.

Conventionally, a laparoscopic surgery or a thoracoscopic surgery is used when performing an intraabdominal or intrathoracic surgery. An instrument called "trocar" as disclosed in, e.g., Patent Documents 2, 3 is used in the laparoscopic and thoracoscopic surgeries. The trocar is formed by a cannula in the shape of a straight tube, and an inner needle that is inserted in the cannula. When using this trocar, a small incision of about 5 mm to 20 mm is first made in the skin. With the inner needle kept inserted in the cannula, the inner needle is pressed into the small incision in the skin, so that the inner needle and the cannula together pass through body surface tissue into an abdominal cavity or a thoracic cavity. Then, the inner needle is removed from the cannula, and a surgical instrument such as a laparoscope, a thoracoscope, forceps, an electrocautery, or a suture instrument is inserted into the cannula, whereby operation procedures can be performed.

Surgeries involving incision and opening of a thoracic part or an abdominal part are also common in addition to the laparoscopic and thoracoscopic surgeries. CITATION LIST

PATENT DOCUMENT
PATENT DOCUMENT 1: Japanese Patent Publication No. 2006-147482
PATENT DOCUMENT 2: Japanese Patent Publication No. H08-168465
PATENT DOCUMENT 3: Japanese Patent Publication No. 2006-87609

### SUMMARY OF THE INVENTION

### TECHNICAL PROBLEM

However, the laparoscopic and thoracoscopic surgeries do not involve opening of a thoracic or abdominal part. Thus, light of lighting equipment installed on the ceiling does not reach the inside of a cavity (a thoracoscopic cavity or an abdominal cavity). Thus, the inside of the cavity can be illuminated only by light of an endoscope inserted into the cavity via a trocar, and sufficient brightness cannot be ensured in a wide range. This makes it difficult for an operator to smoothly perform procedures.

In common surgeries involving opening of the thoracic or abdominal part, light from a shadowless lamp is directed to an operative field. However, since the shadowless lamp merely emits light from above the operative field, the light may not reach deep inside the cavity. This also makes it difficult for the operator to smoothly perform procedures.

The present invention was developed in view of the above problems, and it is an object of the present invention to enable an operative field to be directly illuminated in a wide range when performing a surgery, thereby enabling an operator to smoothly perform procedures.

### SOLUTION TO THE PROBLEM

In order to achieve the above object, in a first invention, a surgical lighting system includes: a lighting portion having a light emitting body; and a fixing portion configured to fix the lighting portion to a living organism to be operated on.

With this configuration, the lighting portion can be fixed to the living organism by the fixing portion. The lighting portion is introduced into, e.g., a thoracic or abdominal cavity via a trocar, and is fixed to biological tissue, whereby the inside of the cavity can be illuminated in a wide range with sufficient brightness.

In common surgeries involving opening of a thoracic or abdominal part, the direction of the light emitting body is determined so that light reaches deep inside the thoracic or abdominal cavity, and the lighting portion is fixed to the biological tissue, whereby the surgical lighting system can illuminate deep inside the cavity with sufficient brightness.

In a second invention, a surgical lighting system includes: a lighting portion having a light emitting body; and an operation portion configured to perform an operation of introducing the lighting portion into a living organism to be operated on.

With this configuration, the lighting portion can be introduced into the living organism by operating the operation portion by an operator. As in the first invention, the inside of the cavity of the living organism can be illuminated by the lighting portion.

In a third invention, a surgical lighting system includes a lighting portion having a light emitting body, and the lighting portion is configured to be placed on a living organism to be operated on.

With this configuration, the surgical lighting system can be used with the lighting portion being placed on the living organism. As in the first invention, the inside of the cavity of the living organism can be illuminated by the lighting portion.

In a fourth invention, in the first invention, the fixing portion is a needle protruding from the lighting portion.

With this configuration, the illuminating portion can be fixed by merely inserting the needle into the biological tissue.

In a fifth invention, in the first invention, a thermal insulating material is provided between the light emitting body of the lighting portion and the fixing portion.

With this configuration, heat of the light emitting body is less likely to be transmitted to the biological tissue.

In a sixth invention, in the first invention, the surgical lighting system further includes a removing member formed to extend to outside of the living organism.

With this configuration, in the case where the lighting portion is located inside the cavity, the removing member extends to the outside of the living organism. Thus, the lighting portion can be easily taken out of the living organism by pulling the removing member. Moreover, since the removing member extends to the outside of the living organism, the removing member also serves as a marker indicating that the lighting portion is still in the cavity.

In a seventh invention, in any one of the first to sixth inventions, the surgical lighting system further includes a guiding tool configured to guide the lighting portion and the fixing portion into a cavity of the living organism.

With this configuration, the lighting portion and the fixing portion are guided into the cavity by the guiding tool when fixing the lighting portion to the inside of the cavity. Thus, the lighting portion can be easily fixed.

In an eighth invention, in any one of the first to seventh inventions, the surgical lighting system contains a battery that is connected to the light emitting body.

With this configuration, electric power can be supplied to the light emitting body by the battery. This eliminates the need for a wiring, a connector, etc. for supplying external electric power.

In a ninth invention, in any one of the first to eighth inventions, the surgical lighting system further includes a shooting portion.

With this configuration, a region illuminated by light of the light emitting body can be shot by the shooting portion.

In a tenth invention, in any one of the first to ninth inventions, the fixing portion is formed by a protecting tool that is fixed to the living organism so as to cover an incised part of the living organism, and the lighting portion is attached to the protecting tool.

With this configuration, the lighting portion can be fixed to the living organism via the protecting tool on the incised part.

In an eleventh invention, in any one of the first to tenth inventions, the light emitting body is formed by a light emitting diode or an organic electroluminescence (EL) device.

With this configuration, a sufficient amount of light can be obtained while making the lighting portion compact. Since the light emitting diode and the organic EL device generate a small amount of heat, the influence of the heat generated by the light emitting body on the biological tissue can be reduced.

### ADVANTAGES OF THE INVENTION

According to the first invention, the surgical lighting system includes: the lighting portion having the light emitting body; and the fixing portion configured to fix the lighting portion to the living organism to be operated on. Thus, in various surgeries, an operative field can be illuminated in a wide range with sufficient brightness, and procedures can be smoothly performed.

According to the second invention, the surgical lighting system includes: the lighting portion having the light emitting body; and the operation portion configured to perform the operation of introducing the lighting portion into the living organism to be operated on. Thus, as in the first invention, the operative field can be illuminated in a wide range with sufficient brightness, and procedures can be smoothly performed.

According to the third invention, the surgical lighting system includes the lighting portion having the light emitting body, and the lighting portion is configured to be able to be placed on the living organism to be operated on. Thus, as in the first invention, the operative field can be illuminated in a wide range with sufficient brightness, and procedures can be smoothly performed.

According to the fourth invention, the fixing portion is formed by the needle. Thus, the lighting portion can be fixed to any position by merely inserting the needle into the biological tissue. This can facilitate the operation of fixing the lighting portion.

According to the fifth invention, the thermal insulating material is provided between the light emitting body of the lighting portion and the fixing portion. Thus, heat of the light emitting body is less likely to be transmitted to the biological tissue, whereby the biological tissue is hardly affected by the heat, and a further minimally invasive treatment can be provided.

According to the sixth invention, the surgical lighting system further includes the removing member formed to extend to the outside of the living organism. Thus, in the case where the lighting portion is located inside the cavity, the lighting portion can be easily taken out of the cavity by pulling the removing member. Moreover, the removing member, which extends to the outside of the living organism, also functions as a marker indicating that the lighting portion is still in the cavity. This can reduce the possibility that the lighting portion may be left in the cavity, whereby safety of the medical practice can be increased.

According to the seventh invention, the surgical lighting system further includes the guiding tool configured to guide the lighting portion and the fixing portion into the cavity. Thus, the lighting portion can be easily introduced into the cavity and fixed to the inside of the cavity, whereby performance of the operation of fixing the lighting portion can be improved.

According to the eighth invention, the surgical lighting system contains the battery. This eliminates the need for a wiring, a connector, etc. for supplying external electric power to the light emitting body of the lighting portion, whereby performance of the operation of fixing the lighting portion to the inside of the cavity can be improved.

According to the ninth invention, a region illuminated by light of the light emitting body can be shot by the shooting portion, whereby a clear image that is useful for procedures can be obtained.

According to the tenth invention, the lighting portion is fixed to the protecting tool that is fixed to the living organism so as to cover the incised part of the living organism. This reduces the risk of damage to the biological tissue, such as scratches, when fixing the lighting portion to the living organism, whereby the lighting portion can be fixed in a minimally invasive manner.

According to the eleventh invention, the light emitting body of the lighting portion is formed by the light emitting diode or the organic EL device. Thus, the lighting portion can be made compact, and the amount of heat that is generated by the light emitting body is reduced, whereby the influence of fixing the lighting portion on the biological tissue can be reduced, and a minimally invasive surgery can be performed.

### BRIEF DESCRIPTION OF THE DRAWINGS

[FIG. 1] FIG. 1 is a perspective view of a lighting device of a first embodiment.
[FIG. 2] FIG. 2 is a front view of the lighting device.
[FIG. 3] FIG. 3 is a longitudinal section of the lighting device.
[FIG. 4] FIG. 4 is a cross-sectional view taken along line IV-IV in FIG. 3.
[FIG. 5] FIG. 5 is a cross-sectional view of a flexible tube and a covering material in a linear state.
[FIG. 6] FIG. 6 is a cross-sectional view of the flexible tube and the covering material in a curved state.
[FIG. 7] FIG. 7 is a cross-sectional view of an abdominal part of a patient punctured by a trocar.
[FIG. 8] FIG. 8 is a diagram corresponding to FIG. 7, showing a state where the lighting device has been inserted into an abdominal cavity by using a holding tool.
[FIG. 9] FIG. 9 is a diagram corresponding to FIG. 7, showing a state where the lighting device has been fixed to biological tissue inside the abdominal cavity.
[FIG. 10] FIG. 10 is a diagram illustrating a state where a plurality of lighting devices have been fixed to the biological tissue inside the abdominal cavity.
[FIG. 11] FIG. 11 is a diagram corresponding to FIG. 3, showing a modification of the first embodiment.
[FIG. 12] FIG. 12 is a diagram corresponding to FIG. 2, showing a second embodiment.
[FIG. 13] FIG. 13 is a right side view of a lighting device of the second embodiment.
[FIG. 14] FIG. 14 is a perspective view of a holding tool of the second embodiment.
[FIG. 15] FIG. 15 is a diagram corresponding to FIG. 7, showing a state where the lighting device of the second embodiment is being inserted into an abdominal cavity by a guiding tool.
[FIG. 16] FIG. 16 is a diagram corresponding to FIG. 9, showing the second embodiment.
[FIG. 17] FIG. 17 is a diagram corresponding to FIG. 2, showing a modification.
[FIG. 18] FIG. 18 is a side view of a lighting device of a third embodiment.
[FIG. 19] FIG. 19 is a cross-sectional view taken along line XIX-XIX in FIG. 18.
[FIG. 20] FIG. 20 is a diagram illustrating a state where the lighting devices of the third embodiment have been fixed to a patient.
[FIG. 21] FIG. 21 is a diagram illustrating a state where the lighting devices of the third embodiment are used.
[FIG. 22] FIG. 22 is a perspective view of a lighting device of a fourth embodiment.
[FIG. 23] FIG. 23 is a diagram corresponding to FIG. 22, showing a modification of the fourth embodiment.
[FIG. 24] FIG. 24 is a diagram illustrating a state where the lighting device of the fourth embodiment is used.
[FIG. 25] FIG. 25 is a diagram illustrating other states where the lighting device of the fourth embodiment is used.
[FIG. 26] FIG. 26 is a diagram illustrating still another state where the lighting apparatus of the fourth embodiment is used.
[FIG. 27] FIG. 27 is a diagram corresponding to FIG. 22, showing a modification of the fourth embodiment.
[FIG. 28] FIG. 28 is a perspective view of a lighting device of a fifth embodiment.
[FIG. 29] FIG. 29 is a perspective view of a lighting device of a sixth embodiment.
[FIG. 30] FIG. 30 is a diagram illustrating a state where the lighting device of the sixth embodiment is used.
[FIG. 31] FIG. 31 is a side view of a lighting device of a seventh embodiment.
[FIG. 32] FIG. 32 is a perspective view of a lighting device of an eighth embodiment.
[FIG. 33] FIG. 33 is a diagram illustrating a state where the lighting device of the eighth embodiment is used.
[FIG. 34] FIG. 34 is a diagram showing an image obtained by shooting an abdominal part by cameras respectively provided in a plurality of lighting devices.

### DESCRIPTION OF EMBODIMENTS

Embodiments of the present invention will be described in detail below with reference to the accompanying drawings. Note that the following description of preferred embodiments is merely exemplary in nature, and is in no way intended to limit the invention, its applications, or uses.

### (First Embodiment)

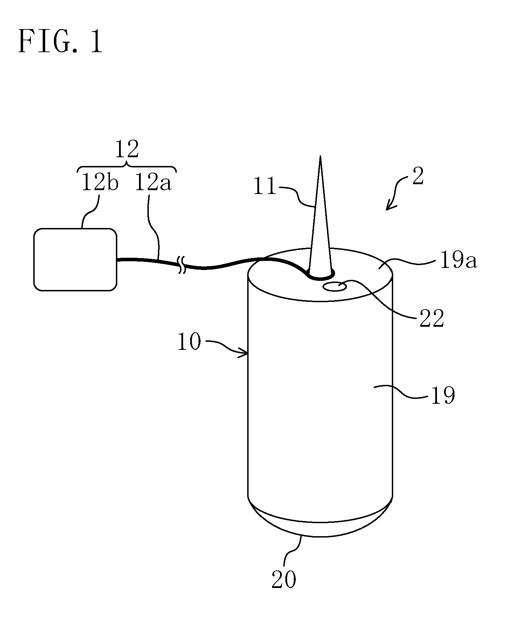

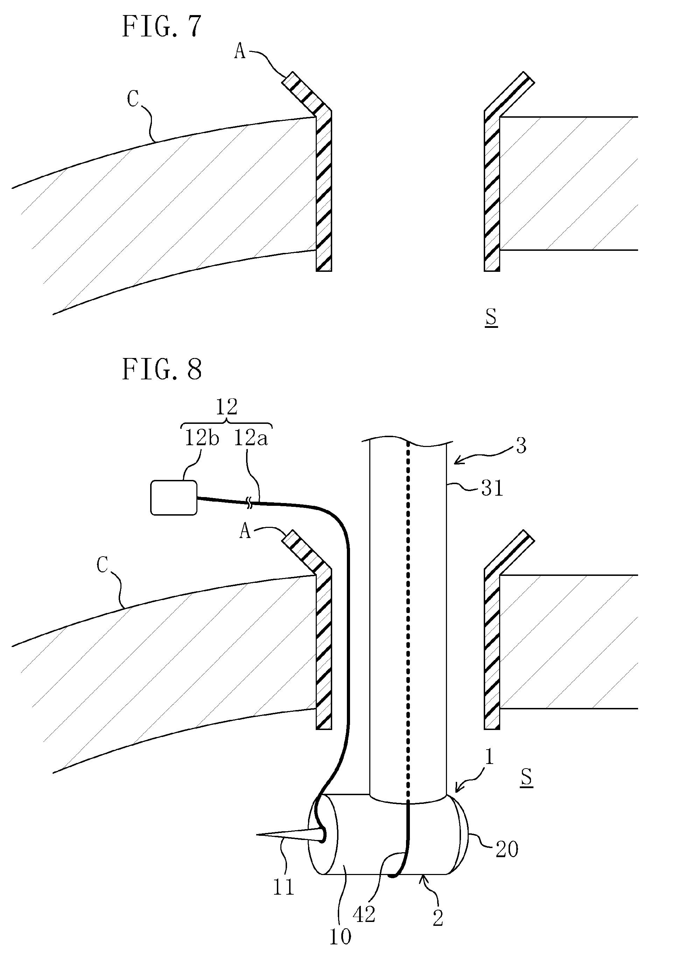

FIG. 1 shows a lighting device 2 that forms a lighting system 1 (shown in FIG. 8) of a first embodiment of the present invention. The lighting system 1 is used in, e.g., laparoscopic surgeries, and includes, in addition to the lighting device 2, a holding tool 3 for holding the lighting device 2.

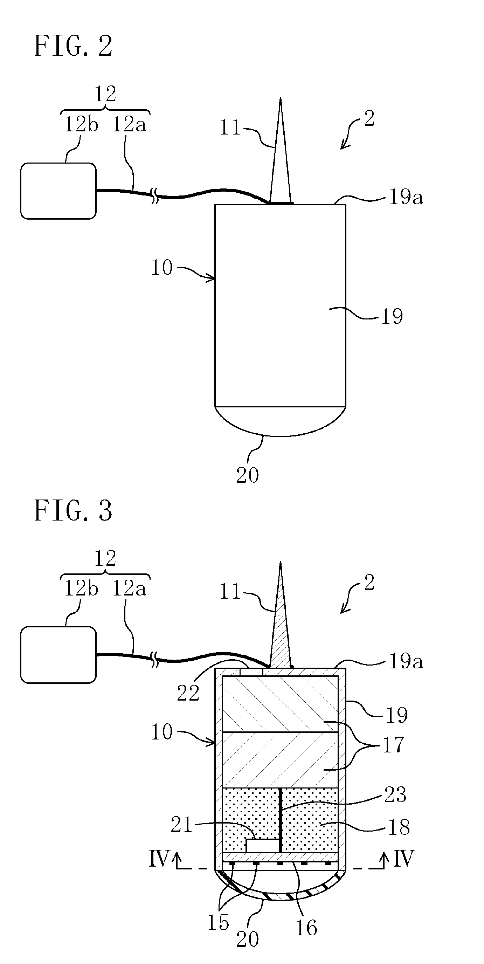

As shown in FIGS. 2-3, the lighting device 2 includes a lighting portion 10, a fixing needle (a fixing portion) 11 for fixing the lighting portion 10 to biological tissue within an abdominal cavity S, and a removing member 12 for taking the lighting portion 10 out of the abdominal cavity S.

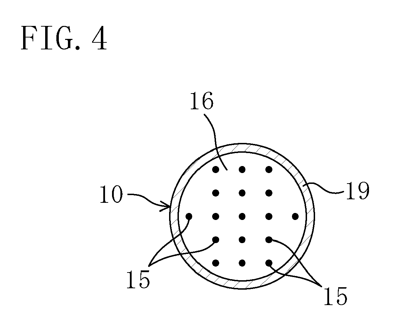

As shown in FIG. 4, the lighting portion 10 includes a light emitting diode (LED) mounting substrate 16 having a plurality of white light emitting diodes (LEDs) 15 mounted thereon, a battery 17 and a thermal insulating material 18, which are shown in FIG. 3, and a case 19 containing the LED mounting substrate 16, the battery 17, and the thermal insulating material 18. The case 19 has a cylindrical shape having an outer diameter of 10 mm or less and an axial dimension of 20 mm or less. Note that the dimensions of the case 19 are not limited to these values.

A member that forms the case 19 is not particularly limited as long as it is made of a material that hardly affects living organisms. A closing plate portion 19a is provided at one longitudinal end of the case 19. A lens 20 is provided at the other longitudinal end of the case 19. The outer face of the lens 20 is curved outward of the case 19. The battery 17 is provided on the side of the closing plate portion 19a in the case 19. The battery 17 is a well-known button cell battery. The life of the battery 17 is such that the LEDs 15 can be in an on state for about 2-5 hours in total. Thus, the lighting portion 10 is of a so-called a disposable type that is discarded after each use.

On the other hand, the LED mounting substrate 16 is provided near the end on the side of the lens 20 in the case 19, and has a disc shape corresponding to the cross-sectional shape of the case 19. As shown in FIG. 4, the LEDs 15 are arranged in a matrix pattern on the face on the side of the lens 20 of the LED mounting substrate 16. The number of LEDs 15 and the positions thereof can be arbitrarily determined other than as illustrated in FIG. 4. For example, a single LED 15 may be provided near the center of the LED mounting substrate 16. In addition to the white LEDs 15, red, green, and blue LEDs may be provided to emit light of a wavelength that makes it easier to observe the biological tissue. An LED that emits near infrared light may be provided. An LED that emits near infrared light may be provided.

The LED mounting substrate 16 is provided with a control circuit 21 that controls electric power to be supplied to the LEDs 15. The battery 17 is connected to the control circuit 21 via a wiring 23. The case 19 is provided with a turn-on switch 22 for starting power supply to the LEDs 15. The turn-on switch 22 is provided to face outward of the case 19, and does not protrude beyond the outer surface of the case 19. The turn-on switch 22 is connected to the control circuit 21. The control circuit 21 is a well-known circuit configured to start power supply to the LEDs 15 upon detection of operation of the turn-on switch 22.

The thermal insulating material 18 is provided between the LED mounting substrate 16 and the battery 17 in the case 19. The thermal insulating material 18 can be made of, but is not limited to, a resin foam material, etc.

A needle 11 protrudes straight outward of the case 19 from the central portion of the closing plate portion 19a of the case 19. The removing member 12 is formed by a string 12a and a small piece member 12b. The base end of the string 12a is tied to the base end of the needle 11. The small piece member 12b is attached to the tip end of the string 12a. The length of the string 12a is, e.g., about 300 mm, so that with the lighting portion 10 being fixed to the biological tissue in the abdominal cavity S, the tip end side of the string 12a extends to the outside, and the small piece member 12b is located outside the abdominal cavity S. Note that a thread, a wire, etc. may be used instead of the string 12a. The shape of the small piece member 12b is not limited to that shown in the figures.

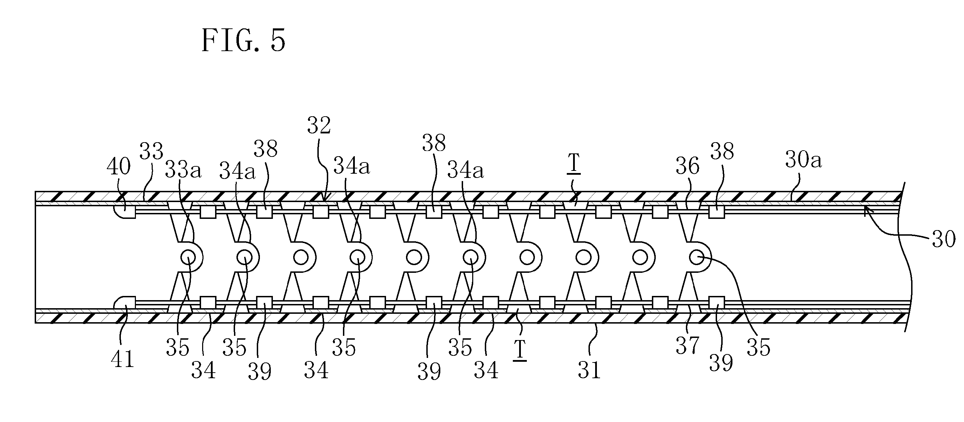

The holding tool 3 includes a flexible tube 30 shown in FIG. 5, a covering member 31 that covers the outer peripheral surface of the flexible tube 30, an operation portion (not shown) for curving the flexible tube 30, and a holding wire 42 (shown in FIG. 8). A curving mechanism 32 formed in a tube shape is provided in a predetermined range on the side of the tip end of the flexible tube 30. A tip end tube portion 33 is provided at the tip end of the curving mechanism 32.

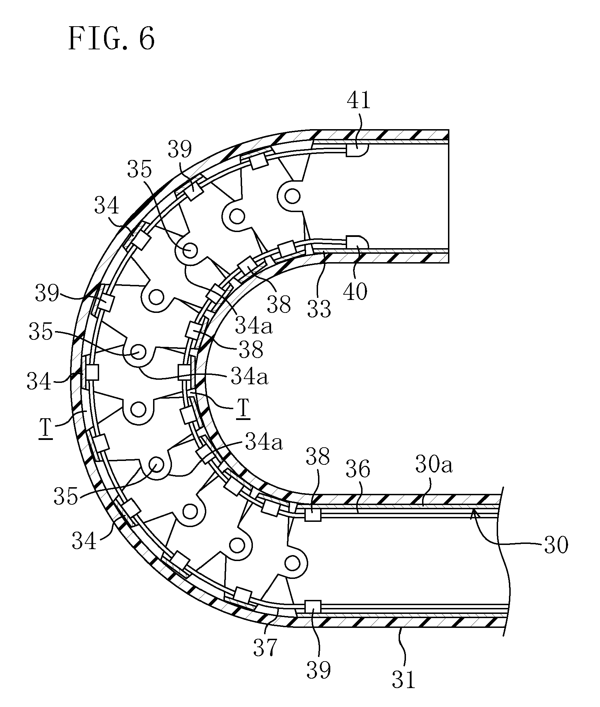

The curving mechanism 32 is formed by arranging a plurality of annular members 34, 34, ... in the direction of the centerline of the flexible tube 30. Two protruding pieces 34a protruding in the direction of the centerline of the flexible tube 30 are formed on a peripheral wall portion of each annular member 34 so as to be separated from each other by about 180° in the circumferential direction. These protruding pieces 34a are coupled to the peripheral wall portion of an adjacent one of the annular members 34 by a pin 35 extending in a direction extending through this peripheral wall portion. Each annular member 34 is configured to pivot about the centerline of the pin 35. The peripheral wall portion of each annular member 34 is tapered so that the dimension in the direction of the centerline is progressively reduced from the protruding pieces 34a toward the inner peripheral surface of the flexible tube 30 in the circumferential direction. Thus, between adjacent ones of the annular members 34, 34 is formed a gap T that is progressively increased in size from the protruding pieces 34a toward the inner peripheral surface of the flexible tube 30 in the circumferential direction. The gap T thus formed between adjacent ones of the annular members 34, 34 allows each annular member 34 to pivot with respect to the annular members 34 located adjacent to that annular member 34. As shown in FIG. 6, the size of the gap T is determined so that the flexible tube 30 is curved until the tip end side of the flexible tube 30 is formed into a "U" shape, namely until the tip end tube portion 33 faces the base end side of the flexible tube 30.

The annular member 34 located at the base end of the flexible tube 30 in the curving mechanism 32 is pivotally coupled to a main body 30a of the flexible tube 30 via the same pin 35 as that described above. The tip end tube portion 33 is also provided with two protruding pieces 33a, and these protruding pieces 33a are pivotally coupled via the pin 35 to the annular member 34 located at the tip end of the curving mechanism 32.

As shown in FIG. 5, a first operation wire 36 and a second operation wire 37 are arranged inside the flexible tube 30 so as to extend substantially parallel to the centerline from the tip end to the base end of the flexible tube 30. The first operation wire 36 and the second operation wire 37 are separated from each other by 180° in the circumferential direction of the flexible tube 30, and are separated from the protruding pieces 34a of the annular members 34 by 90° in the circumferential direction. The base ends of the first and second operation wires 36, 37 are coupled to a pair of take-up shafts of the operation portion, respectively.

Hollow first and second wire guides 38, 39, which are configured to receive the first and second operation wires 36, 37 therethrough, respectively, are provided on the inner peripheral surface of each annular member 34 at positions corresponding to those where the operation wires 36, 37 are provided. The first and second wire guides 38, 39 are similarly provided on the inner peripheral surface of the main body 30a of the flexible tube 30. First and second wire fixing members 40, 41, to which the tip ends of the first and second operation wires 36, 37 are fixed, respectively, are also provided on the inner peripheral surface of the tip end tube portion 33 at positions corresponding to those where the operation wires 36, 37 are provided.

The curving mechanism 32 is curved as shown in FIG. 6 by taking up the first operation wire 36 by the take-up shaft of the operation portion and loosening the second operation wire 37. On the other hand, the curving mechanism 32 is curved to the opposite side by loosening the first operation wire 36 and taking up the second operation wire 37 by the take-up shaft. The degree to which the curving mechanism 32 is curved can be arbitrarily adjusted by the amount by which the first and second operation wires 36, 37 are taken up.

The covering material 31 is made of a resin material having flexibility, and is formed to closely contact the outer peripheral surface of the flexible tube 30 from the base end to the tip end thereof. The flexible tube 30 is integral with the covering material 31. The degree of flexibility of the covering material 31 is determined so as not to hinder the curving operation of the curving mechanism 32 of the flexible tube 30.

The holding wire 42 is configured to hold the lighting device 2, and is inserted through the flexible tube 30. The tip end of the holding wire 42 forms a ring, and protrudes beyond the tip end of the flexible tube 30. The lighting device 2 is held by the tip end of the holding wire 42. The base end of the holding wire 42 protrudes beyond the base end of the flexible tube 30 so that an operator can pull the base end of the holding wire 42.

An example of using the lighting system 1 configured as described above will be described below. First, as shown in FIG. 7, an abdominal part C of a patient to be operated on is punctured by trocars A. The number of trocars A is four. Then, carbon dioxide is introduced into the abdominal cavity S via the trocars A to inflate the abdominal part C.

Then, the lighting device 2 is held by the holding wire 42 of the holding tool 3. That is, the ring portion at the tip end of the holding wire 42 is placed around the outer periphery of the case 19 of the lighting device 2, and the base end of the holding wire 42 is pulled, whereby the lighting device 2 is sandwiched and held between the ring portion of the holding wire 42 and the tip end of the flexible tube 30. The turn-on switch 22 of the lighting device 2 is on at this time.

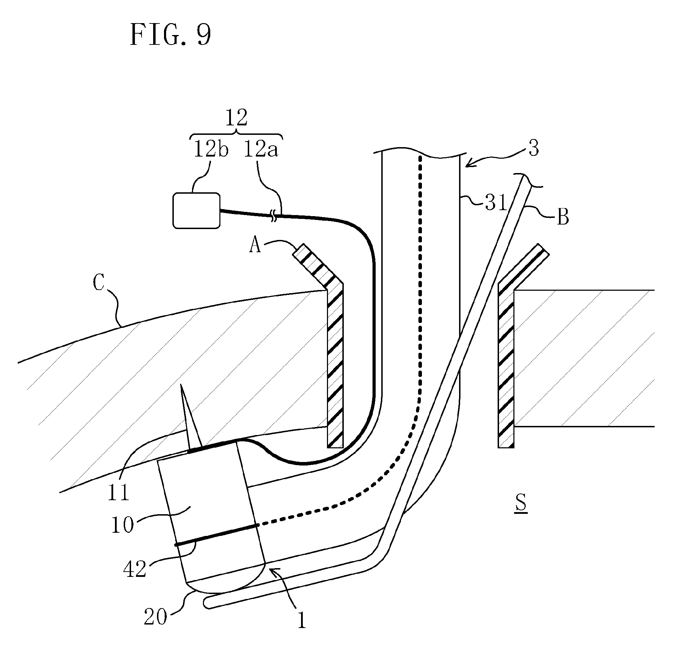

Thereafter, as shown in FIG. 8, the lighting device 2 is inserted into the abdominal cavity S via the trocar A. At this time, since the direction of the lighting device 2 can be changed by reducing the holding force of the holding wire 42, the lighting device 2 is oriented to such a direction that the lighting device 2 can be easily inserted into the trocar A. That is, the lighting device 2 is oriented so that the axial direction of the lighting device 2 is substantially the same as the axial direction of the trocar A. After inserting the lighting device 2 into the abdominal cavity S in this manner, as shown in FIG. 9, the flexible tube 30 is curved to change the direction of the lighting device 2 so that the tip end of the needle 11 is inserted in the biological tissue. When inserting the lighting device 2, the tip end side of the removing member 12 is located outside of the trocar A.

A pressing tool B for pressing the lighting device 2 against the biological tissue is inserted into the abdominal cavity S through the trocar A, and the tip end of the pressing tool

B is placed on the lighting device 2 to press the lighting device 2 against the biological tissue. Thus, the needle 11 is inserted in the biological tissue and is fixed thereto.

Thereafter, the holding wire 42 is loosened, whereby the flexible tube 30 is removed through the trocar A, and the pressing tool B is also removed through the trocar A.

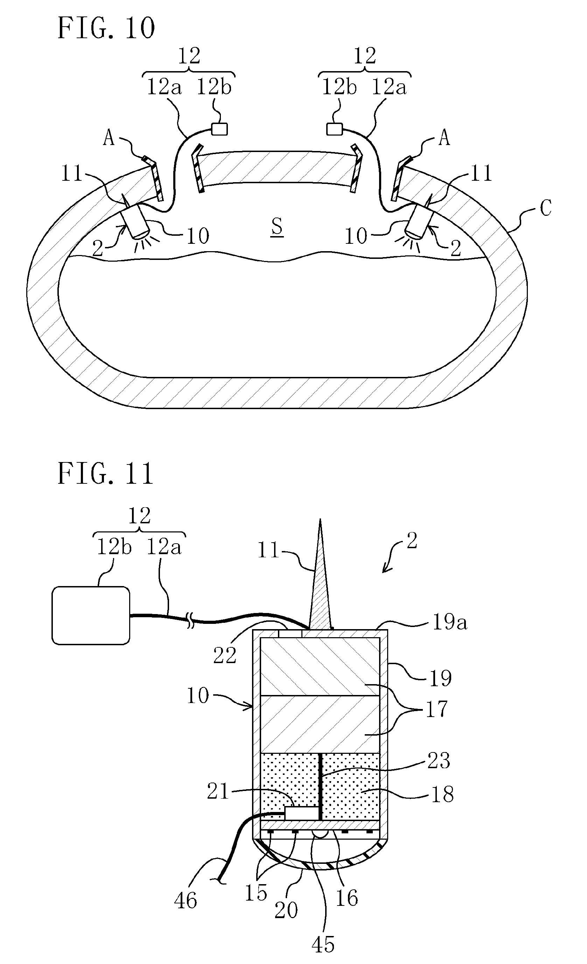

As shown in FIG. 10, the lighting device 2 can be inserted into the abdominal cavity S through another one of the trocars A and fixed to the biological tissue. Thus, the lighting device 2 can be provided at a plurality of positions in the abdominal cavity S.

Since the inside of the abdominal cavity S is illuminated by the lighting device 2 thus fixed to the inside of the abdominal cavity S, sufficient brightness can be ensured in a wide range, and endoscopic observation can be reliably and rapidly performed.

During illumination by the lighting device 2, heat is generated by the LEDs 15. However, since the amount of the heat is significantly smaller than that generated by a light bulb having a filament, etc., the biological tissue is hardly adversely affected by the heat. Moreover, since the thermal insulating material 18 is interposed between the LEDs 15 and the needle 11, the heat of the LEDs 15 is hardly transmitted to the needle 11. This also can prevent the biological tissue from being adversely affected by the heat.

When finishing the operation, the lighting device 2 is grasped and pulled by, e.g., forceps, etc. to remove the needle 11 from the biological tissue and take the needle 11 out of the abdominal cavity S through the trocar A. At this time, since the lighting device 2 is provided with the removing member 12, the needle 11 can be easily taken out of the abdominal cavity S by pulling the removing member 12. Since the removing member 12 extends to the outside through the trocar A, the removing member 12 serves as a marker indicating that the lighting device 2 is still in the abdominal cavity S, whereby the possibility can be reduced that the lighting device 2 may be left in the abdominal cavity S. Note that the lighting device 2 is discarded after being taken out of the abdominal cavity S. Moreover, holding the removing member 12 can prevent the lighting device 2 from dropping in the abdominal cavity S.

As described above, according to the first embodiment, the lighting portion 10 having the LEDs 15 is fixed to the biological tissue in the abdominal cavity S by the needle 11. Thus, the inside of the abdominal cavity S can be illuminated in a wide range with sufficient brightness, whereby procedures can be smoothly performed.

The lighting portion 10 can be fixed at any position by merely inserting the needle 11 into the biological tissue. This can facilitate the operation of fixing the lighting portion 10.

Since the LEDs 15 are used for illumination, the lighting portion 10 capable of providing sufficient brightness can be made compact, and the amount of heat that is generated by the lighting portion 10 can be reduced. Thus, the influence of fixing the lighting portion 10 on the biological tissue can be reduced, whereby a minimally invasive surgery can be performed.

The thermal insulating material 18 is provided between the LEDs 15 of the lighting portion 10 and the needle 11. This makes it more difficult for the heat generated by the LEDs 15 to be transmitted to the biological tissue. Thus, the biological tissue is hardly affected by the heat, whereby a further minimally invasive treatment can be provided.

The removing member 12 formed to extend to the outside of the living organism is provided. Thus, in the case where the lighting portion 10 is fixed to the inside of the abdominal cavity S, the lighting portion 10 can be easily taken out of the abdominal cavity S by pulling the removing member 12. Moreover, since the removing member 12 extends to the outside of the living organism, the removing member 12 functions as a marker indicating that the lighting portion 10 is still in the abdominal cavity S. This can reduce the possibility that the lighting portion 10 may be left in the abdominal cavity S, whereby safety of the medical practice can be increased.

Since the holding tool 3 for holding the lighting portion 2 is provided, the lighting device 2 can be easily held and introduced into the abdominal cavity S by the holding tool 3.

The lighting device 2 contains the battery 17. This eliminates the need for a wiring, a connector, etc. for supplying external electric power to the LEDs 15 of the lighting portion 10, whereby performance of the operation of fixing the lighting device 2 to the inside of the peritoneal cavity S can be improved.

By fixing the plurality of lighting devices 2 to the inside of the abdominal cavity S, a region to be observed can be illuminated from multiple directions, whereby a shadow is less likely to be created on the region to be observed.

As in a modification shown in FIG. 11, a camera (a shooting portion) 45 may be attached to the lighting device 2. The lighting device 2 is provided with a signal line 46 for transmitting a signal of an image captured by the camera 45 to an image display device, not shown. The use of this lighting device 2 enables the inside of the abdominal cavity S to be shot while being illuminated, whereby a clear image that is useful for procedures can be obtained. The angle of view of the camera 45 is preferably about 50°-80°, and the number of pixels is preferably about 3-5 millions. However, the present invention is not limited to these ranges. The camera 45 is preferably of a type that can also be used in infrared applications. The camera 45 is preferably of a scan type using a charge-coupled device (CCD). However, the present invention is not limited to this.

Note that although the lighting system 1 includes the holding tool 3 in the present embodiment, the holding tool 3 may be omitted. The lighting system 1 may be configured by placing the plurality of lighting devices 2, 2. In this case, the lighting devices 2, 2 may have different brightness values and different light wavelengths from each other.

### (Second Embodiment)

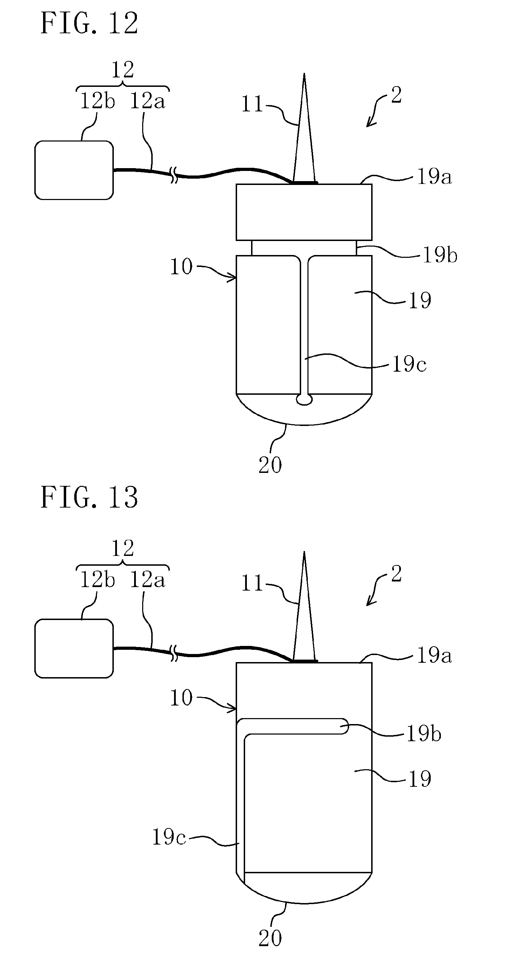

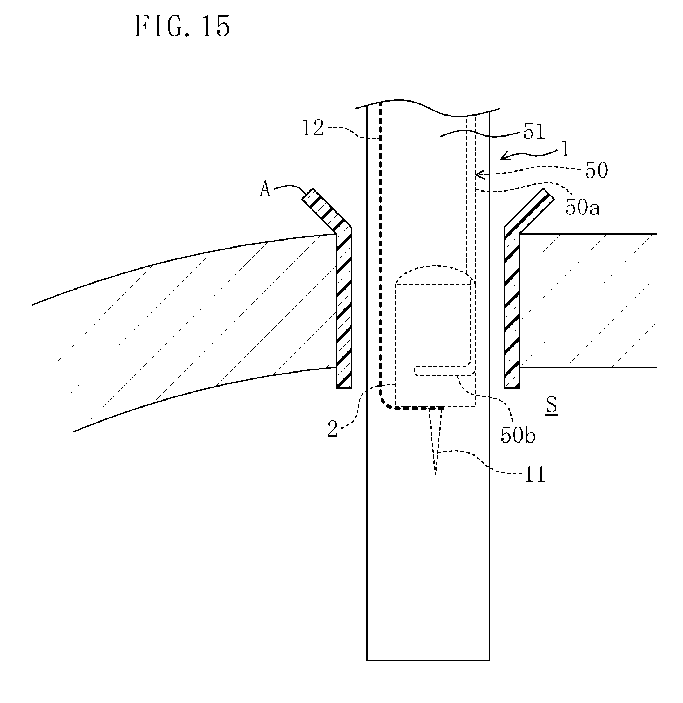

FIG. 12 shows a lighting device 2 of a second embodiment of the present invention. A lighting system 1 (shown in FIG. 15) of the second embodiment is different from the first embodiment in the structure of a holding tool 50 for holding the lighting device 2 and in that the lighting system 1 includes a guiding tool 51. In the following description, the same portions as those of the first embodiment are denoted with the same reference characters, and description thereof is omitted. Those portions different from the first embodiment will be described in detail below.

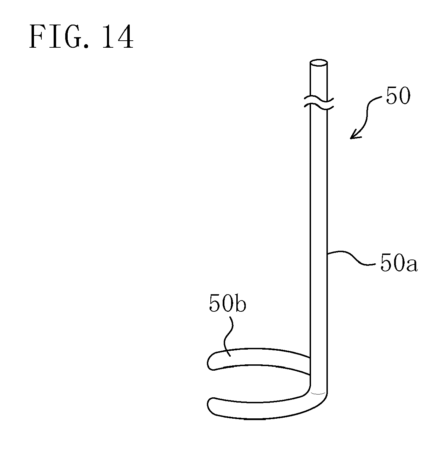

As shown in FIG. 14, the holding tool 50 of the second embodiment includes a main body 50a formed by a bar member, and a C-shaped portion 50b provided at the tip end of the main body 50a. The circumferential center of the C-shaped portion 50b is fixed to the main body 50a. The main body 50a and the C-shaped portion 50b are made of a flexible, soft resin.

As shown in FIGS. 12-13, grooves 19b, 19c are formed in the outer surface of the case 19 of the lighting device 2. The groove 19b extends in the circumferential direction so that the C-shaped portion 50b of the holding tool 50 is fitted therein. The groove 19c extends in the axial direction of the case 19 continuously with the longitudinal center of the groove 19b so that the main body 50a is fitted therein. The groove 19b is located on the side close to the needle 11 of the case 11, and the groove 19c extends to the lens 20. The lighting device 2 is held by the holding tool 50 by fitting the C-shaped portion 50b of the holding tool 50 in the groove 19b, and fitting the main body 50a in the groove 19c. At this time, the needle 11 protrudes to the side opposite to the main body 50a of the holding tool 50.

The guiding tool 51 shown in FIG. 15 is formed by a flexible tube, a covering material, and an operation portion (all of which are not shown), which are configured similarly to the flexible tube 30, the covering material 31, and the operation portion of the holding tool 3 of the first embodiment. The inner diameter of the flexible tube is larger than the outer diameter of the lighting device 2, so that the lighting device 2 oriented to extend in the axial direction of the flexible tube can be inserted through the flexible tube.

An example of using the lighting system 1 of the second embodiment will be described below with reference to FIGS. 15-16. First, the guiding tool 51 is inserted into the abdominal cavity S through the trocar A, and the lighting device 2 is held by the holding tool 50. An operator holds the main body 50a of the holding tool 50 to insert the lighting device 2 into the flexible tube.

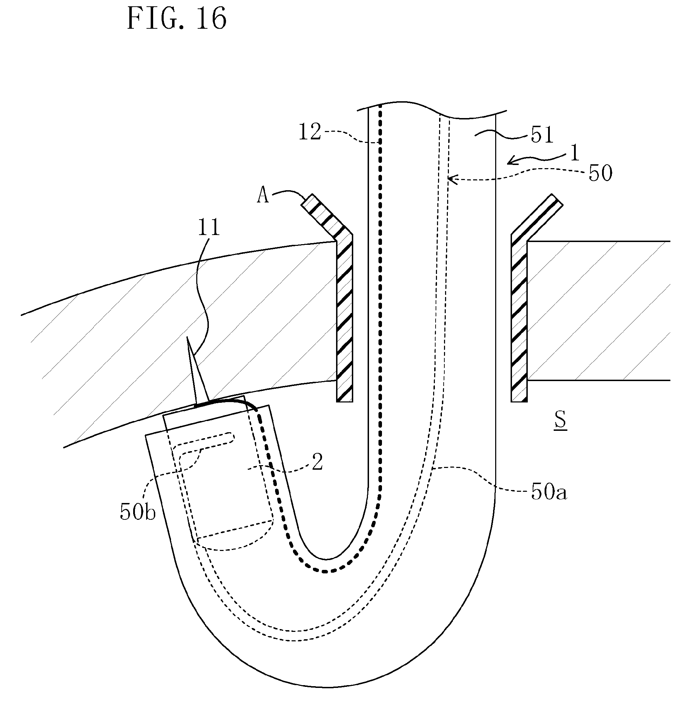

Then, as shown in FIG. 16, the flexible tube is curved so that the tip end of the flexible tube faces the biological tissue, and the main body 50a of the holding tool 50 is pressed in the insertion direction of the flexible tube. Thus, the pressure is transmitted to the lighting device 2, and the needle 11 of the lighting device 2 is inserted in the biological tissue. The lighting device 2 is fixed in this manner. At this time, the removing member 12 extends to the outside from the base end of the flexible tube.

After fixing the lighting device 2, the guiding tool 51 is removed through the trocar A. Then, e.g., forceps, etc. are inserted into the abdominal cavity S through the trocar A. Thus, the main body 50a and the C-shaped portion 50b of the holding tool 50 are grasped and removed from the grooves 19b, 19c of the lighting device 2, and the holding tool 50 is removed through the trocar A.

Thus, according to the second embodiment, as in the first embodiment, the inside of the abdominal cavity S can be illuminated in a wide range with sufficient brightness, and procedures can be smoothly performed.

Since the guiding tool 51 for guiding the lighting device 2 into the abdominal cavity S is provided, the lighting device 2 can be easily introduced into the abdominal cavity S, and performance of the operation of fixing the lighting device 2 can be improved.

The lighting devices 2 of the first and second embodiments can be similarly fixed to a thoracic cavity when used in a thoracoscopic surgery.

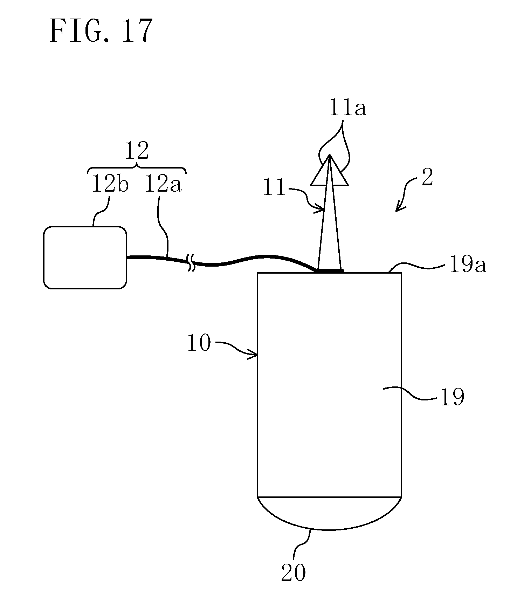

Note that as in a modification shown in FIG. 17, a retaining portion 11a may be provided at the tip end of the needle 11 of the lighting devices 2 of the first and second embodiments. This retaining portion 11a is shaped to protrude in the radial direction from the tip end of the needle 11, so that the retaining portion 11a bites into the biological tissue with the needle 11 inserted therein. Thus, the needle 11 is prevented from coming off.

### (Third Embodiment)

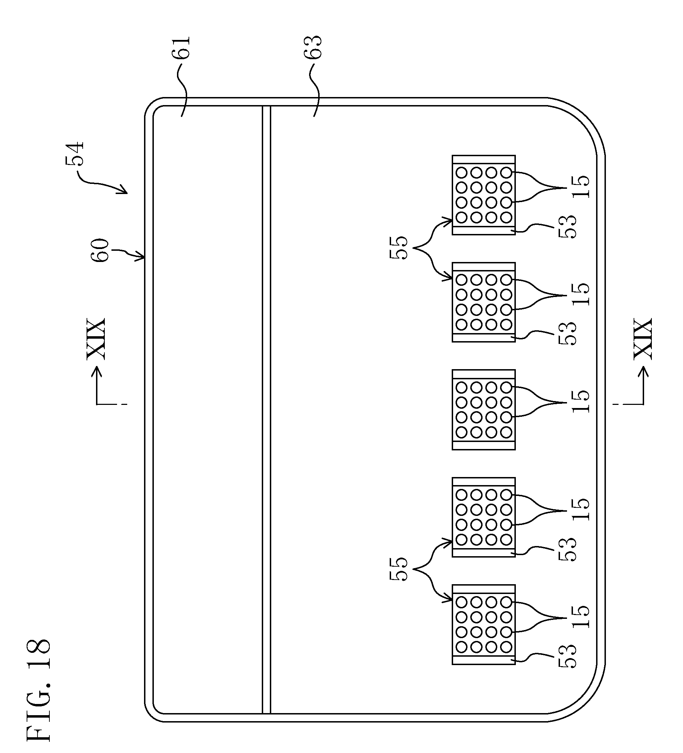

FIG. 18 shows a lighting system 1 of a third embodiment of the present invention. A lighting device 54 of the third embodiment is different from the first and second embodiments in that lighting portions 55 are attached to a protecting tool 60 for protecting an incised part of a patient C. In the following description, the same portions as those of the first and second embodiments are denoted with the same reference characters, and description thereof will be omitted. Those portions different from the first and second embodiments will be described in detail below.

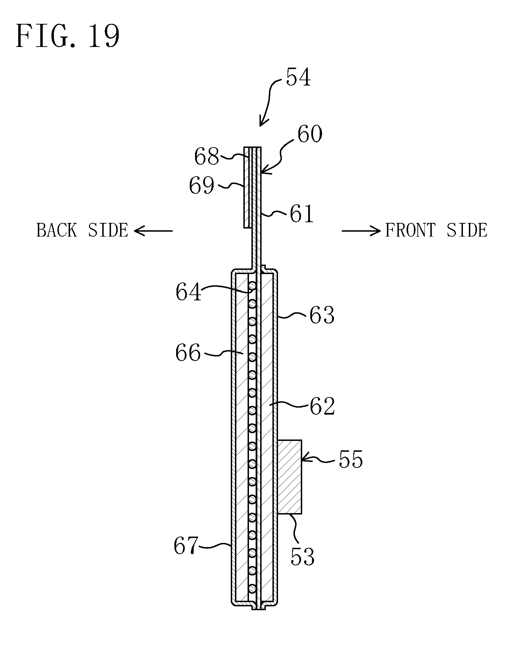

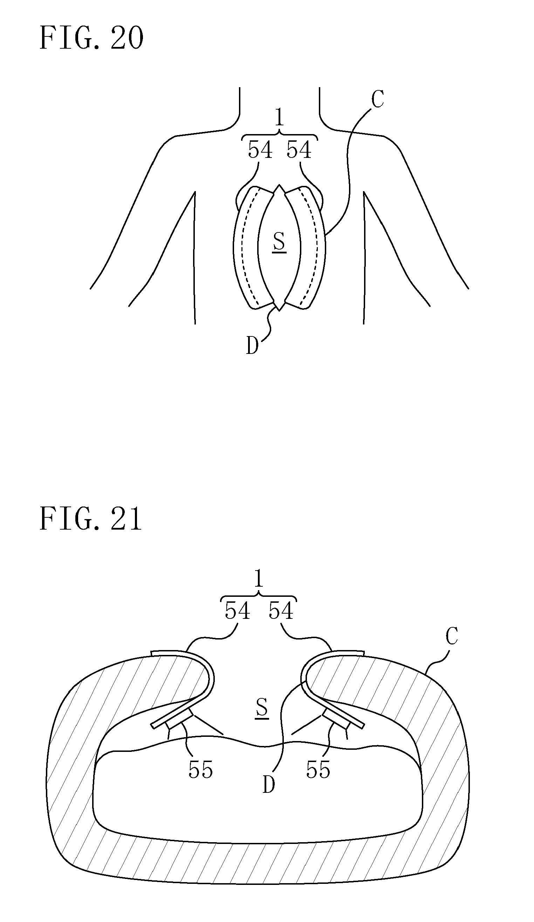

As shown in FIG. 20, the lighting system 1 of the present embodiment is formed by a pair of right and left lighting devices 54. Each lighting device 54 includes the lighting portions 55 and the protecting tool (a fixing portion) 60. As shown in FIG. 19, each lighting portion 55 has no needle, and is configured to be bonded to the front side of the protecting tool 60. Each lighting device 54 includes cases 53 having a rectangular box shape, and a rectangular LED mounting substrate (not shown) having LEDs 15 mounted thereon, a control circuit (not shown), and a battery (not shown) are provided in each case 53. Each case 53 is provided with a turn-on switch (not shown).

The protecting tool 60 is a tool for protecting an incised part D (shown in FIG. 20) formed by making an incision in body surface tissue when operating on a thoracic or abdominal part. As shown in FIG. 19, the protecting tool 60 includes a resin intermediate sheet 61, a first liquid absorbing material 62 provided on the front side of the intermediate sheet 61, a first cloth material 63 holding the first liquid absorbing material 62 between the first cloth material 63 and the intermediate sheet 61, a base material 64 placed on the back side of the intermediate sheet 61, a second liquid absorbing material 66, and a second cloth material 67 holding the second liquid absorbing material 66. An adhesive 68 and a backing sheet 69 that covers the adhesive 68 are provided at a position away from the base material 64 on the back side of the second cloth material 67.

The intermediate sheet 61 is formed by a rectangular semitransparent film made of polyethylene. The longitudinal dimension of the intermediate sheet 61 is about 210 mm, and the lateral dimension thereof is about 150 mm. As shown in FIG. 18, the four corners of the intermediate sheet 61 are formed by curves. Note that the intermediate sheet 61 may be made of polyurethane, polyvinyl chloride, etc., or may be made of a film having a multilayer structure formed by stacking these resin materials together.

The first liquid absorbing material 62 is made of water swellable fibers. LANSEAL made by TOYOBO CO., LTD., which is formed by an inner layer of acrylic fibers and an outer layer of a water absorbing resin, can be used as the water swellable fibers. The rate of water absorption of the water swellable fibers is such that the water swellable fibers absorb about 50% or more of equilibrium water absorption in about 10 seconds when in contact with water. After absorbing water, the water swellable fibers do not release the water even if pressed to some degree. The water swellable fibers are insoluble in water. Moreover, the diameter of the water swellable fibers after absorbing water is at least about five times before absorbing water. On the other hand, the longitudinal dimension of the fibers is maintained by the acrylic fibers, and is almost the same before and after absorbing water. Physical properties of the water swellable fibers are maintained by the acrylic fibers, and thus are hardly degraded even if the water absorbing resin of the outer layer absorbs water. Note that the first liquid absorbing material 62 may be made of gauze made of cotton, rayon, etc., or may be made of nonwoven fabric formed by mixing cotton or rayon with the water swellable fibers, or a stacked body formed by stacking the water swellable fibers on cotton or rayon.

The first cloth material 63 is made of nonwoven fabric having water permeability. The nonwoven fabric that forms the first cloth material 63 has a heat seal property so that the nonwoven fabric is welded to a resin material by heating.

The adhesive 68 is an acrylic, silicone, polyurethane, or rubber adhesive that is commonly used for application to human skin.

The backing sheet 69 is a sheet produced by performing a releasing process on a resin sheet, paper, etc. with a silicone release agent, etc. For example, a polyethylene terephthalate film, a polypropylene film, etc. can be used when forming the backing sheet 69 from the resin sheet. Glassine paper, clay coated paper, laminated paper, etc. can be used when forming the backing sheet 69 from the paper.

The base material 64 is formed by combining a multiplicity of resin wires in a mesh pattern, and has substantially the same shape as the first liquid absorbing material 62 as viewed in plan. Each wire is made of a resin material that is not broken when being bent, and has a shape retaining property so that the wire material retains the bent shape. For example, polyethylene, polypropylene, polyester, nylon, etc. can be used as the resin material. Polyethylene having the highest shape retaining property among these resin materials is used in the present embodiment.

The second liquid absorbing material 66 and the second cloth material 67 are the same as the first liquid absorbing material 62 and the first cloth material 63, respectively.

Although not shown in the figures, when stored, the protecting tool 60 is contained together with sterile paper in a bag made of a resin film having no moisture permeability. This can prevent degradation in liquid absorption capability of the first and second liquid absorbing materials 62, 63 due to absorption of moisture in the air while being stored.

An example of using the lighting system 1 of the present embodiment will be described below. As shown in FIGS. 20-21, after opening an incised part D, the protecting tool 60 is oriented so that the second cloth material 67 faces the incised part D. Then, the protecting tool 60 is bent along the side edge of the incised part D to cover the side edge of the incised part D. Thereafter, the backing sheet 69 is removed from the adhesive 68, and a sticking portion 68 is applied the skin so as to closely contact the surface of the skin. Thus, the protecting tool 60 is fixed to the patient. The lighting portions 55 are placed in the thoracic cavity or the abdominal cavity S. The directions of the lighting portions 55 are determined so that light from the LEDs 15 reaches deep inside the thoracic cavity or the abdominal cavity S. Note that the number of lighting portions 55 may be arbitrarily determined.

As described above, the side edge of the incised part D is covered by the protecting tool 60. This makes it difficult for pathogenic bacteria, etc. to adhere to the incised part D, thereby reducing the risk of contagion or infection.

Blood, exudate, etc., coming out of the incised part D during a surgery pass through the second cloth material 67 and are absorbed by the second liquid absorbing material 66.

Thus, according to the third embodiment, when performing a common surgery involving opening of a thoracic or abdominal part, the directions of the LEDs 15 are determined so that light reaches deep inside the thoracic cavity or the abdominal cavity S, and the lighting portions 55 are fixed to the biological tissue. Accordingly, the thoracic cavity and the abdominal cavity S can be illuminated in a wide range with sufficient brightness, and procedures can be smoothly performed.

Since the lighting portions 55 are attached to the protecting tool 60, the needle 11 need not be inserted in the biological tissue as in the first and second embodiments, whereby the lighting portions 55 can be fixed in a minimally invasive manner.

The lighting device 54 of the third embodiment may be combined with the lighting devices 2 of the first and second embodiments to configure a lighting system. The lighting device 54 of the third embodiment may be provided with such a camera as in the modification of the first embodiment.

### (Fourth Embodiment)

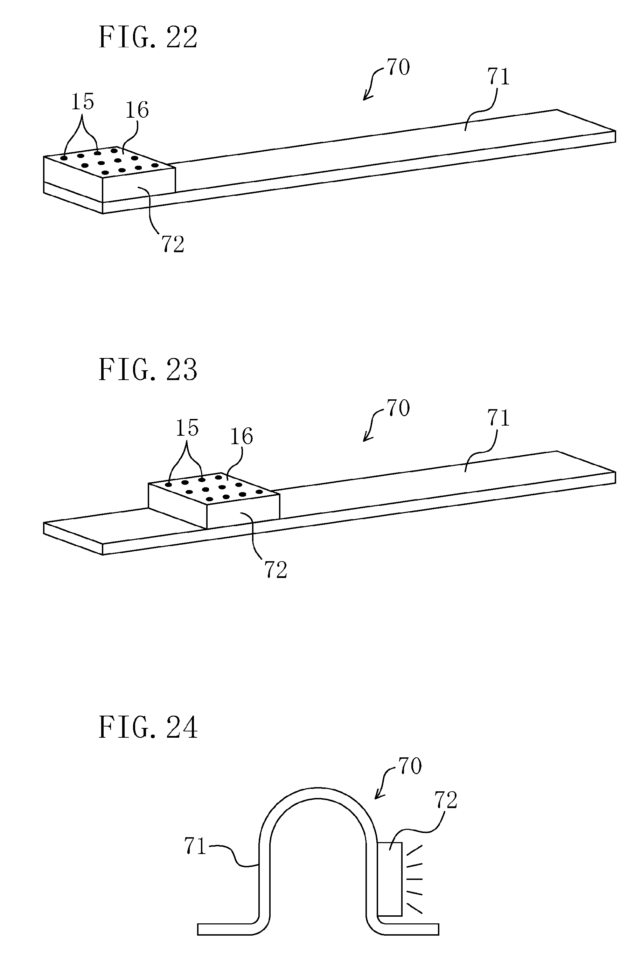

FIG. 22 shows a lighting device 70 according to a fourth embodiment of the present invention. The lighting device 70 is different from the first embodiment in that a lighting portion 72 is attached to a plate member 71. In the following description, the same portions as those of the first embodiment are denoted with the same reference characters, and description thereof will be omitted. Those portions different from the first embodiment will be described in detail below.

The lighting device 70 includes the lighting portion 72 and the plate member 71. The lighting portion 72 has a box shape. Although not shown in the figures, the lighting portion 72 contains an LED mounting substrate, a battery, and a thermal insulating material. The LED mounting substrate is placed to face the surface of the lighting portion 72 so that the LEDs 15 emit light in a direction outward of the lighting portion 72.

The plate member 71 can be made of, e.g., any material that hardly affects living organisms, such as stainless steel, an aluminum alloy, etc. This plate member 71 has such a rigidity that allows an operator to bend the plate member 71 by hand, and retains the bent shape. The thickness of the plate member 71 is preferably, e.g., about 0.5 mm to 2.0 mm. However, the present invention is not limited to this.

The lighting portion 72 is bonded to the surface of the plate member 71. As in a modification shown in FIG. 23, the lighting portion 72 may be bonded to an intermediate portion in the longitudinal direction of the plate member 71. The position to which the lighting portion 72 is bonded may be arbitrarily determined.

Heat generated by the lighting portion 72 is conducted to the plate member 71 and dissipated. That is, the plate member 71 also functions also as a heat dissipating member of the lighting portion 72.

When using the lighting device 70, the plate member 71 is bent as shown in, e.g., FIG. 24 to form a horizontal portion at both longitudinal ends of the plate member 71, and to curve the intermediate portion in the longitudinal direction of the plate member 71 upward. After making an incision in the abdominal part of the patient, the lighting device 70 is placed into the abdominal cavity, and the horizontal portions of the plate member 71 are placed on the biological tissue inside the abdominal cavity. Thus, the lighting device 70 is deployed inside the abdominal cavity, and the inside of the abdominal cavity is illuminated by light of the lighting portion 72. The direction in which the lighting portion 72 emits light can be arbitrarily determined according to the shape of the plate member 71.

As described above, according to the fourth embodiment, the lighting portion 72 having the LEDs 15 can be deployed inside the abdominal cavity. Thus, the inside of the abdominal cavity can be illuminated in a wide range with sufficient brightness, and procedures can be smoothly performed.

The plate member 71 may have a multilayer structure in which a core is made of a metal material such as stainless steel, an aluminum alloy, etc., and is coated with a resin material. The plate member 71 may be made of a shape memory alloy.

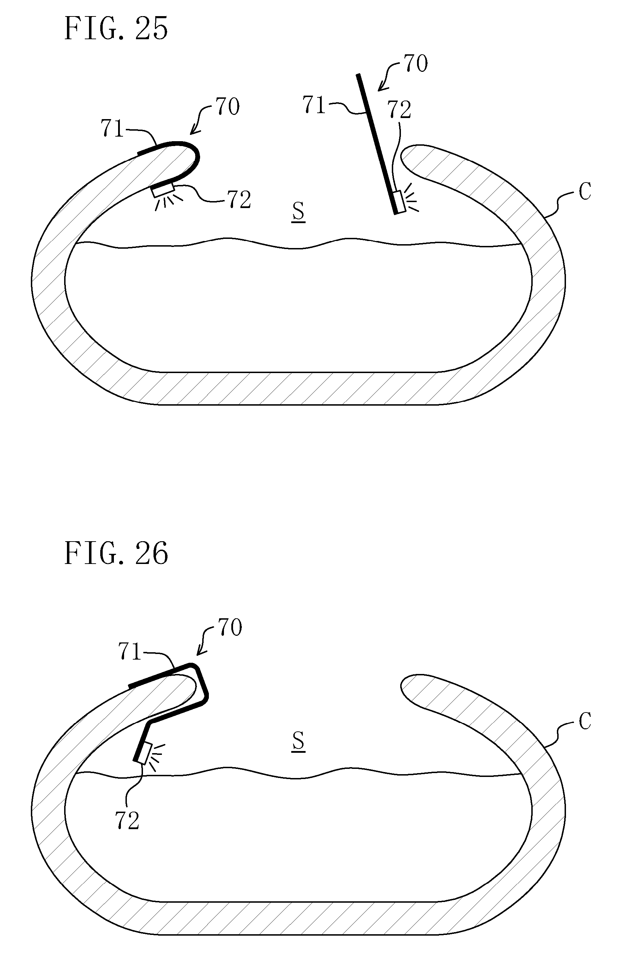

As shown on the left side of FIG. 25, the plate member 71 may be bent to extend from the surface side of the abdominal part C through the opening edge into the abdominal cavity S, so that the lighting portion 72 is fixed to a position near the opening edge in the abdominal cavity S. In this case, the plate member 71 serves as a fixing portion.

As shown on the right side of FIG. 25, the plate member 71 may be used without bending. The plate member 71 serves as a handle for an operator or an assistant to hold, and the operator or the assistant can hold the plate member 71 to introduce the lighting portion 72 into the abdominal cavity S.

As shown in FIG. 26, the plate member 71 may be bent at three positions. The plate member 71 may be bent at two positions, or at four or more positions. Alternatively, the plate member 71 may be curved.

The plate member 71 may be provided with an adhesive member for living organisms. Examples of the adhesive member include a double sided adhesive tape, etc. When using the double sided adhesive tape, it is preferable that a corner portion of a backing be separated from the plate member 71 in order to make it easier to peel off the backing. Alternatively, an adhesive may be applied to the plate member 71.

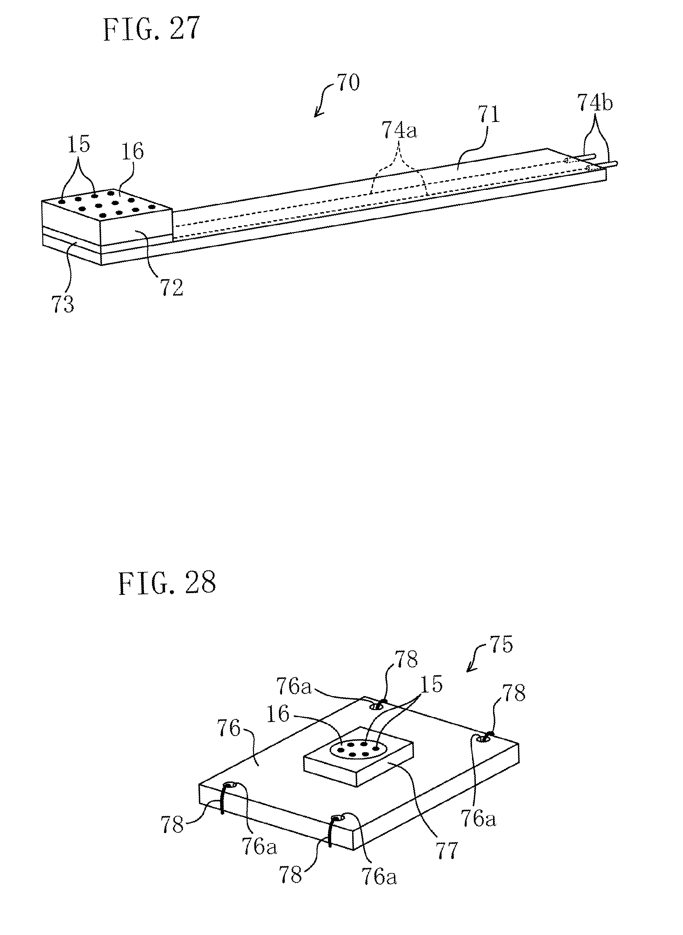

As in a modification shown in FIG. 27, a thermal insulating material 73 may be provided between the lighting portion 72 and the plate member 71. Thus, heat generated by the lighting portion 72 is not directly conducted to the plate member 71, whereby the influence of the heat on the patient can be reduced. In this modification, wirings 74a, 74a extending from the lighting portion 72 are embedded in the plate member 71. The wirings 74a, 74a extend to the opposite end of the plate member 71 from the lighting portion 72. Connector pins 74b, 74b are respectively provided at the opposite ends of the wirings 74a, 74a from the lighting portion 72. An external power supply unit is connected to the connector pins 74b, 74b.

### (Fifth Embodiment)

FIG. 28 is a lighting device 75 of a fifth embodiment of the present invention. The lighting device 75 is different from the first embodiment in that a lighting portion 77 is attached to a fixing plate (a fixing portion) 76. In the following description, the same portions as those of the first embodiment are denoted with the same reference characters, and description thereof will be omitted. Those portions different from the first embodiment will be described in detail below.

The lighting device 75 includes the lighting portion 77 and the fixing plate 76. Although not shown in the figure, the lighting portion 77 contains an LED mounting substrate, a battery, and a thermal insulating material as in the fourth embodiment.

The fixing plate 76 is produced by forming a material similar to that of the plate member 71 of the fourth embodiment into a rectangular shape. A plurality of through holes 76a, 76a, ... are formed at intervals in the circumferential direction in the periphery of the fixing plate 76. The through holes 76a are configured to receive a surgical thread 78 therethrough. Note that the fixing plate 76 may have, e.g., a circular shape.

The lighting portion 77 is bonded to the surface of the fixing plate 76. The lighting portion 77 may be bonded to a position near the periphery of the fixing plate 76. The position to which the lighting portion 77 is bonded may be arbitrarily determined.

When using the lighting device 75, an incision in made in the abdominal part of the patient, and the lighting device 75 is then placed in the abdominal cavity to place the fixing plate 76 on the biological tissue inside the abdominal cavity. Then, the thread 78 is inserted through each through hole 76a of the fixing plate 76 to sew the fixing plate 76 on the biological tissue. Thus, the lighting device 75 is fixed, and the inside of the abdominal cavity is illuminated by light of the lighting portion 77. The direction in which the lighting portion 77 emits light can be arbitrarily determined according to the location to which the fixing plate 76 is fixed.

As described above, according to the fifth embodiment, the lighting portion 77 having the LEDs 15 can be deployed inside the abdominal cavity. Thus, the inside of the abdominal cavity can be illuminated in a wide range with sufficient brightness, and procedures can be smoothly performed.

Like the plate member 71 of the fourth embodiment, the fixing plate 76 may be provided with an adhesive member for living organisms, which is formed by a double sided adhesive tape, etc. Alternatively, an adhesive may be applied to the fixing plate 76.

### (Sixth Embodiment)

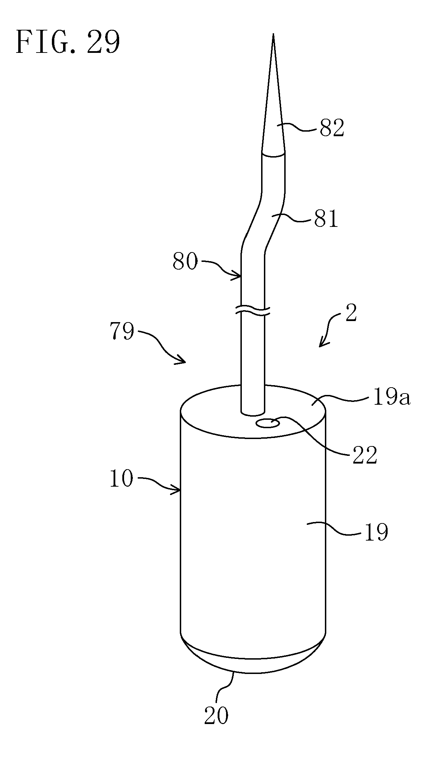

FIG. 29 shows a lighting device 79 according to a sixth embodiment of the present invention. The lighting device 79 is different from that of the first embodiment in the fixing structure of the lighting portion 10. In the following description, the same portions as those of the first embodiment are denoted with the same reference characters, and description thereof will be omitted. Those portions different from the first embodiment will be described in detail below.

The lighting device 79 includes a fixing portion 80 formed by a wire 81 and a needle 81, and the lighting portion 10 of the first embodiment. The base end of the wire 81 is fixed to the closing plate portion 19a of the case 19. The needle 82 is fixed to the tip end of the wire 81. The length of the wire 81 is preferably 100 mm or more. The wire 81 is preferably made of stainless steel, etc.

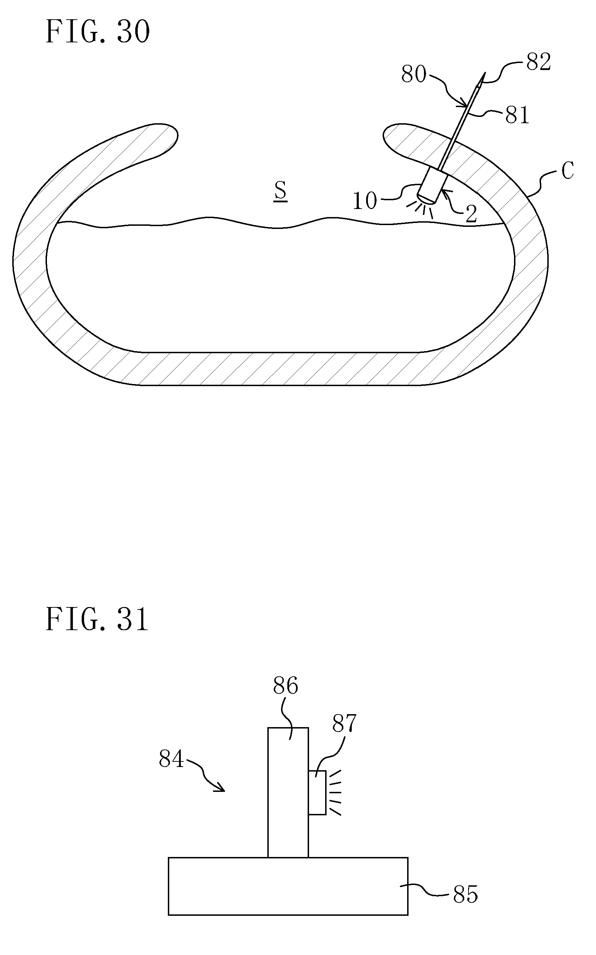

When using the lighting device 79, as shown in FIG. 30, an incision is made in the abdominal part C of the patient, the lighting device 79 is then placed in the abdominal cavity S, and the needle 82 is inserted in the biological tissue from the abdominal cavity S toward the body surface so as to extend through the biological tissue. Thereafter, the needle 28 is separated from the wire 81 on the body surface side. Although not shown in the figures, a knot is tied in the wire 81 on the body surface side. This knot prevents the wire 81 from coming off, whereby the lighting portion 10 is fixed, and the inside of the abdominal cavity S is illuminated by light of the lighting portion 10. Instead of tying a knot in the wire 81, the wire 81 may be nipped and held by forceps, etc.

As described above, according to the sixth embodiment, the lighting portion 10 having the LEDs 15 can be fixed to the inside of the abdominal cavity S. Thus, the inside of the abdominal cavity S can be illuminated in a wide range with sufficient brightness, and procedures can be smoothly performed.

Note that a thread or a string may be used instead of the wire 81.

### (Seventh Embodiment)

FIG. 31 shows a lighting device 84 according to a seventh embodiment of the present invention. The lighting device 84 is different from that of the first embodiment in that a lighting portion 87 is attached to a base 85. The same portions as those of the first embodiment are denoted with the same reference characters, and description thereof will be omitted. Those portions different from the first embodiment will be described in detail below.

The lighting device 84 includes the lighting portion 87 and the base 85. The base 85 is produced by forming a material similar to that of the plate member 71 of the fourth embodiment into a thick plate shape, and has an attachment member 86 to which the lighting portion 87 is attached. The lighting portion 87 can be attached to any position on the attachment member 86.

When using the lighting device 84, an incision is made in the abdominal part of the patient, and the lighting device 84 is then placed into the abdominal cavity to place the base 85 on the biological tissue inside the abdominal cavity. Thus, the lighting device 84 is deployed, and the inside of the abdominal cavity is illuminated by light of the lighting portion 87. The direction in which the lighting portion 87 emits light can be arbitrarily determined according to the location where the base 85 is placed.

As described above, according to the seventh embodiment, the lighting portion 87 having the LEDs 15 can be deployed inside the abdominal cavity. Thus, the inside of the abdominal cavity can be illuminated in a wide range with sufficient brightness, and procedures can be smoothly performed.

### (Eighth Embodiment)

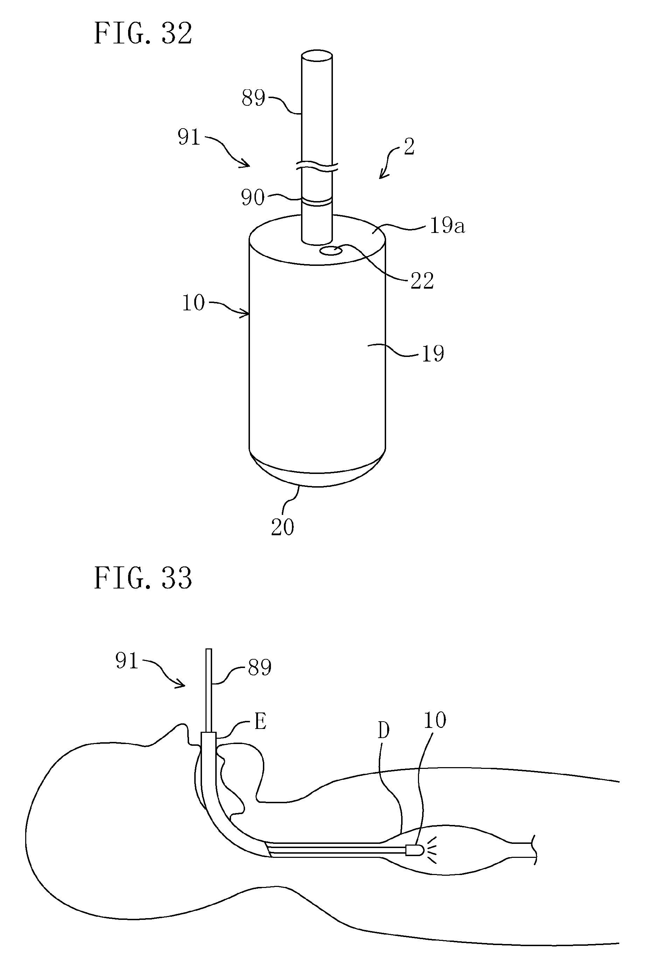

FIG. 32 shows a lighting device 91 of an eighth embodiment of the present invention. The lighting device 91 includes a bar member 89 as an operation portion, and the lighting portion 10 of the first embodiment. The base end of the bar member 89 is detachably fixed to the closing plate portion 19a of the case 19 via a detachable mechanism 90. The length of the bar member 89 is preferably 500 mm or more. The bar member 89 is flexible, and is used to perform an operation of introducing the lighting portion 10 into the body of the patient to be operated on.

The lighting device 91 is used to observe a digestive organ by using an endoscope, or to operate on a digestive organ by using an endoscope. As shown in FIG. 33, when using the lighting device 91, a guide tube E is first inserted toward an esophagus through a patient's mouth. Then, an operator holds the bar member 89 to insert the lighting portion 10 into the guide tube E, and introduce the lighting portion 10 into a stomach D as a digestive organ. The position of the lighting portion 10 can be easily changed by operating the bar member 89. The lighting portion 10 can also illuminate the inside of the esophagus.

As described above, according to the eighth embodiment, the lighting portion 10 having the LEDs 15 can be introduced to the digestive organ. Thus, the inside of the digestive organ can be illuminated in a wide range with sufficient brightness, and procedures can be smoothly performed.

Although not shown in the figure, the control circuit 21 of each lighting device 2 may be provided with a battery level detecting portion for detecting the battery level of the battery 17, a transmitter/receiver, and a control section for controlling brightness of the LEDs 15. The transmitter/receiver is configured to be able to wirelessly communicate with the transmitter/receiver of another lighting device 2. The communication form of the transmitter/receiver is not particularly limited, and for example, visible light communication may be used.

The transmitter/receiver is configured to transmit a detection signal when reduction in battery level is detected by the battery level detecting portion. Thus, when a plurality of lighting devices 2 are used, and for example, the battery level of one of the plurality of lighting devices 2 is reduced, such reduction in battery level is detected by the battery level detecting portion, and a battery level reduction signal is transmitted from the transmitter/receiver to the remainder of the lighting devices 2. Each lighting device 2 that has received the battery level reduction signal increases the brightness of the LEDs 15 by the control section. Thus, the remainder of the lighting devices 2 can compensate for reduction in brightness of the lighting device 2 having the reduced battery level. Moreover, an alarm portion may be provided which gives an alarm such as light, sound, etc. when the battery level is reduced.

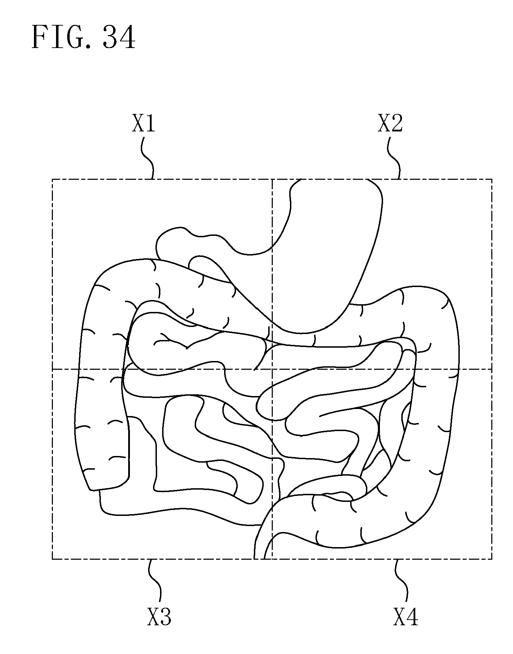

In the case where the control circuit 21 includes the transmitter/receiver, a signal of an image captured by the camera 45 can be wirelessly transmitted to an external image display device (not shown) and displayed thereon. The image display device is configured to receive image signals of the cameras 45 of the plurality of lighting devices 2. The image display device is provided with an image processing section for processing the images captured by the cameras 45. The image processing section is configured to synthesize the images captured by the cameras 45 into an extensive image for output. The image thus produced is shown in FIG. 34. In the figure, a region X1 represents an image of an upper right region of the patient's abdominal part captured by the camera 45, a region X2 represents an image of an upper left region of the abdominal part captured by the camera 45, and similarly, regions X3, X4 represent images of lower right and lower left regions of the abdominal part captured by the cameras 45, respectively. The regions actually captured by the cameras 45 are larger than the regions X1-X4, and overlapping portions are deleted when synthesizing the images. As shown in the figure, a stomach, a small intestine, a large intestine can be seen at a time. The regions X1-X4 may have different sizes from each other. The thoracic part may be shot by the cameras 45.

The image processing section can also output the images captured by the cameras 45 as they are without synthesizing them.

Note that the LEDs 15 may be provided on the outer peripheral surface of the case 19. In this case, a wider range can be illuminated.

Although the LEDs 15 are provided in the first to eighth embodiments, the present invention is not limited to this, and a light emitting body such as a light bulb or an organic electroluminescense (EL) device may be provided, or any combination thereof may be used. The type of the light emitting body is not limited to those described above. An external power supply unit may be connected to the light emitting body.

Since the shape of the light emitting plane can be arbitrarily designed in the organic EL device, the organic EL device may be formed in the same shape as, e.g., the end face of the case 19 to secure a large light emitting plane. Alternatively, the organic EL device may be shaped to extend along the outer peripheral surface of the case 19 so that light can be emitted from the outer peripheral surface of the case 19.

The surgical lighting system may be configured only by the lighting device 2, 54, 70, 75, 79, 84, 91.

Note that the above methods of using the surgical lighting system are merely exemplary, and the lighting system may be used by other methods.

### INDUSTRIAL APPLICABILITY

As described above, the lighting system of the present invention can also be used for surgeries involving incision and opening of a thoracic or abdominal part, such as a laparoscopic surgery and a thoracoscopic surgery.

### DESCRIPTION OF REFERENCE CHARACTERS

- 1: Surgical Lighting System
- 10: Lighting Portion
- 11: Needle (Fixing Portion)
- 12: Removing Member
- 15: LED
- 17: Battery
- 18: Thermal Insulating Material
- 45: Camera (Shooting Portion)
- 51: Guiding Tool
- 60: Protecting Tool
- 85: Base
- 89: Bar Member (Operation Portion)
- A: Trocar
- D: Incised Part
- S: Abdominal Cavity

## Claims

1. A surgical lighting system, comprising:
a lighting portion having a light emitting body; and
a fixing portion configured to fix the lighting portion to a living organism to be operated on.

2. A surgical lighting system, comprising:
a lighting portion having a light emitting body; and
an operation portion configured to perform an operation of introducing the lighting portion into a living organism to be operated on.

3. A surgical lighting system, comprising:
a lighting portion having a light emitting body, wherein
the lighting portion is configured to be placed on a living organism to be operated on.

4. The surgical lighting system of claim 1, wherein
the fixing portion is a needle protruding from the lighting portion.

5. The surgical lighting system of claim 1, wherein
a thermal insulating material is provided between the light emitting body of the lighting portion and the fixing portion.

6. The surgical lighting system of claim 1, further comprising:
a removing member formed to extend to outside of the living organism.

7. The surgical lighting system of any one of claims 1 to 6, further comprising:
a guiding tool configured to guide the lighting portion and the fixing portion into a cavity of the living organism.

8. The surgical lighting system of any one of claims 1 to 7, wherein
the surgical lighting system contains a battery that is connected to the light emitting body.

9. The surgical lighting system of any one of claims 1 to 8, further comprising:
a shooting portion.

10. The surgical lighting system of any one of claims 1 to 9, wherein
the fixing portion is formed by a protecting tool that is fixed to the living organism so as to cover an incised part of the living organism, and the lighting portion is attached to the protecting tool.

11. The surgical lighting system of any one of claims 1 to 10, wherein
the light emitting body is formed by a light emitting diode or an organic electroluminescence device.
